(19) Europäisches Patentamt
European Patent Office
Office européen des brevets

(11) Veröffentlichungsnummer: **0 403 963 A2**

# (12) EUROPÄISCHE PATENTANMELDUNG

(21) Anmeldenummer: **90111287.0**

(22) Anmeldetag: **15.06.90**

(51) Int. Cl.[5]: **C07H 15/252, A61K 31/70, C12P 19/56, //(C12P19/56, C12R1:465)**

(30) Priorität: **20.06.89 DE 3920062**

(43) Veröffentlichungstag der Anmeldung: **27.12.90 Patentblatt 90/52**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hedtmann, Udo, Dr.**
**Liederbacher Strasse 6**
**D-6230 Frankfurt am Main(DE)**
Erfinder: **Fehlhaber, Hans-Wolfram, Dr.**
**Thomas-Mann-Strasse 5a**
**D-6270 Idstein(DE)**
Erfinder: **Sukatsch, Dieter-Andreas, Prof. Dr.**
**Heimchenweg 11**
**D-6230 Frankfurt am Main(DE)**
Erfinder: **Kraemer, Hans Peter, Dr.**
**Birkenweg 16**
**D-3550 Marburg(DE)**
Erfinder: **Hoffmann, Dieter, Dr.**
**Feuerdornweg 12**
**D-3550 Marburg(DE)**

(54) **Neue Anthracyclin-Derivate, ein Verfahren zu ihrer Herstellung und ihre Verwendung als Arzneimittel.**

(57) Die vorliegende Erfindung betrifft Anthracyclin-Derivate der Formel I

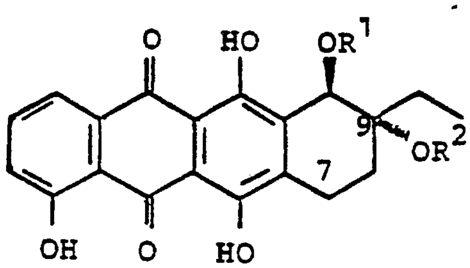

(I)

worin $R^1$ den Zuckerrest Roa oder die Zuckerkombination Roa-Rod-Rod und $R^2$ den Zuckerrest Roa bedeuten, wobei Roa für Rhodosamin der Formel II

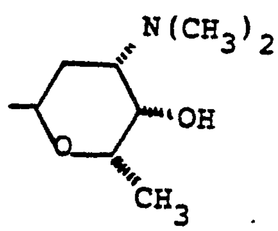

(II)

und Roa-Rod-Rod für Rhodosamin-Rhodinose-Rhodinose der Formel III

EP 0 403 963 A2

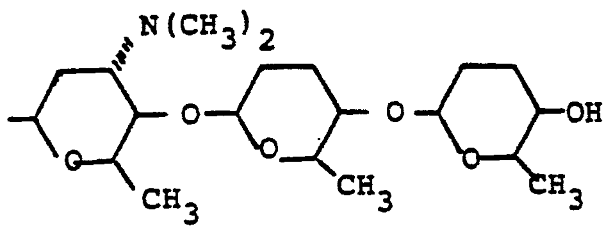

(III)

steht, sowie deren pharmakologisch unbedenkliche Salze, und ein mikrobiologisches Verfahren zu ihrer Herstellung.

Die Verbindungen sind cytostatisch wirksam und können daher als Arzneimittel verwendet werden.

Fig. 1

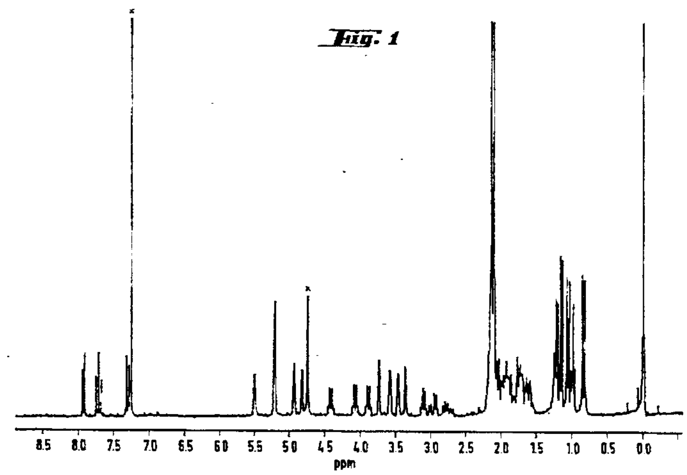

**Neue Anthracyclin-Derivate, ein Verfahren zu ihrer Herstellung und ihre Verwendung als Arzneimittel.**

Gegenstand der vorliegenden Erfindung sind Anthracyclin-Derivate der Formel I

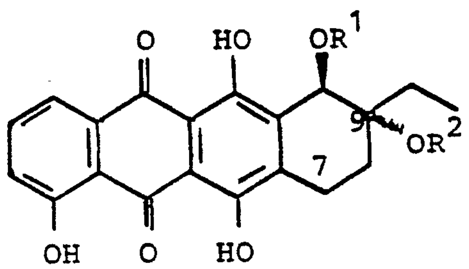

(I)

worin $R^1$ den Zuckerrest Roa oder die Zuckerkombination Roa-Rod-Rod und $R^2$ den Zuckerrest Roa bedeuten, wobei Roa für Rhodosamin der Formel II

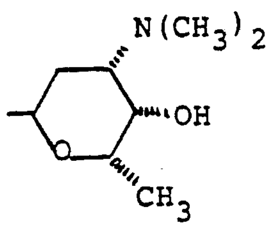

(II)

und Roa-Rod-Rod für Rhodosamin-Rhodinose-Rhodinose der Formel III

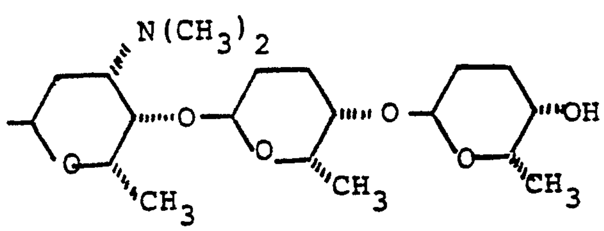

(III)

steht, sowie deren pharmakologisch unbedenkliche Salze.

Gegenstand der Erfindung ist ferner ein Verfahren zur Herstellung der Verbindungen der Formel I, das dadurch gekennzeichnet ist, daß man das Cytorhodin-Rohgemisch, das man als Fraktion B gemäß Beispiel 4 der EP-A-0 131 181 durch Kultivierung des Mikroorganismus-Stammes Y-11 472 (DSM 2658) und Extraktion des Mycels und des Kulturfiltrates mit verschiedenen organischen Lösungsmitteln erhält,

a) in einem wäßrigen Puffer, vorzugsweise bei pH 3,0 - 4,0 löst, die erhaltene Lösung bei stufenweise erhöhten pH-Werten, vorzugsweise bei pH 6,0; 7,0 und 8,0 mit einem organischen Lösungsmittel, vorzugsweise Essigsäureethylester extrahiert, aus den aufkonzentrierten Extrakten durch chromatographische Reinigung eine Verbindung der Formel I erhält, worin $R^1$ Roa-Rod-Rod und $R^2$ Roa bedeutet, und

b) gegebenenfalls die erhaltene Verbindung durch Hydrolyse mit einer anorganischen Säure in eine Verbindung der Formel I, worin $R^1$ und $R^2$ jeweils Roa bedeuten, überführt.

Die Hydrolyse wird mit einer verdünnten anorganischen Säure, vorzugsweise Salzsäure, z.B. unter mehrstündigem Rühren bei Raumtemperatur, durchgeführt. Danach wird die gewünschte Verbindung durch Extraktion z.B. mit Chloroform aus dem Reaktionsmedium gewonnen.

Die Substanzklasse der Anthracyclinonglykoside ist seit langem bekannt. Zu ihnen gehören die cytostatisch wirksamen Verbindungen Daunorubicin und Doxorubicin (vgl. z.B. F. Arcamone, Doxorubicin, Academic Press, Hew York, 1981), die insbesondere zur Behandlung von malignen Tumoren eingesetzt werden. Eine Vielzahl weiterer Vertreter dieser Substanzklasse wurden inzwischen auf biologischem oder

semisynthetischem Weg hergestellt, darunter auch die wegen ihrer überlegenen cytostatischen Wirkung besonders interessanten Cytorhodine (EP-A-0 131 181; EP-A-0 131 942).

Strukturelle Merkmale fast aller biosynthetisch hergestellten Anthracycline sind das unterschiedlich substituierte Anthracyclinongerüst, ein oder mehrere glykosidisch an sekundäre Hydroxygruppen (meist C-7, C-10) gebundene Zucker sowie häufig eine freie, tertiäre OH-Gruppe an C-9 (D.G. Strauss, Pharmazie **42** (1987), 289).

Überraschenderweise konnten nun auch Anthracyclinon-C-9-Glykoside, die sich durch ihre cytostatische Wirksamkeit und geringe akute Toxizität auszeichnen, auf mikrobiologischem Wege hergestellt werden.

Die cytostatische Wirksamkeit wurde an L1210 Leukämiezellen der Maus im sogenannten Proliferationstest wie folgt bestimmt:

L 1210 Zellen in exponentieller Wachstumsphase (5 x $10^3$/ml in RPMI 1640) werden in einer Mikrotiterplatte 72 Stunden mit unterschiedlichen Konzentrationen der Testsubstanz inkubiert (37 °C, 5 % $CO_2$, 95 % relative Luftfeuchtigkeit).

Kontrollen bestehen aus Zellen, die lediglich mit frischem Medium inkubiert werden. Alle Bestimmungen werden als 4-fache Bestimmung durchgeführt. Nach 65 Stunden werden 50 µl MTT (3-(4,5-Dimethylthiazol-2-yl)-2,5-diphenyl-tetrazoliumbromid, 2,5 mg/ml in PBS) zugegeben. MTT wird durch lebende Zellen zu einem roten, unlöslichen Formazan-Farbstoff reduziert. Nach einer weiteren 7-stündigen Inkubation wird das überstehende Medium vorsichtig abgesaugt. Der Formazan-Farbstoff wird durch Zugabe von jeweils 100 µl DMSO in die Vertiefungen der Mikrotiterplatte und vorsichtiges Schütteln gelöst. Die Extinktion der jeweiligen Lösung wird mit Hilfe eines Multiscan Photometers 340 CC, Fa. Flow, bei 492 nm gemessen.

Aus den so erhaltenen Meßwerten wird die Dosiswirkungskurve ermittelt und graphisch die $IC_{50}$, d.h. die Konzentration, die unter den Testbedingungen die Bildung des Formazan-Farbstoffes um 50 % gegenüber der Kontrolle erniedrigt, ermittelt.

Zur Ermittlung der akuten Toxizität wurden $BDF_1$-Mäusen am Tag 0, Tag 3 und Tag 6 unterschiedliche Dosen der Testsubstanz, gelöst in 0,4 ml physiologischer Kochsalzlösung, intravenös injiziert. Kontrollgruppen erhielten lediglich 0,4 ml physiologischer Kochsalzlösung. Pro Konzentration der Testsubstanz wurden 2 Mäuse verwendet. Am Tag 20 wurde die Zahl der überlebenden Mäuse und daraus die $LD_{50}$ ermittelt. Die Toxizität der hier beschriebenen Verbindungen im Vergleich zu Adriamycin ist in Tabelle 1 zusammengefaßt.

Tabelle 1

| Verbindung | Zytotoxizität (Dauerinkubation) $IC_{50}$ (µg/ml) | akute Toxizität $LD_{50}$ (mg/kg) |
|---|---|---|
| Cytorhodin Z | 0,33 | 10 - 25 |
| 9-0,10-0-Bis (α-L-rhodosaminyl)-γ-rhodomycinon | 0,35 | 25 - 50 |
| Adriamycin | 0,02-0,03 | 10 |

Aufgrund der gezeigten cytostatischen Wirksamkeit sind die erfindungsgemäßen Verbindungen zur Behandlung von malignen Tumoren von Säugetieren, z.B. von Gastro-intestinal-, Mamma-, Ovarial- und Bronchialtumoren des Menschen geeignet.

Zur Erläuterung des erfindungsgemäßen Verfahren werden nachstehende Beispiele aufgeführt:

**Beispiel 1**

21 g Cytorhodin-Rohgemisch werden in 5,0 l Natriumacetatpuffer pH 3,0 gelöst, der pH wird anschließend mit 2 N Natriumhydroxidlösung auf 6,0 eingestellt. Die wäßrige Phase wird mit 1 l Ethylacetat extrahiert, mit 2 n NaOH auf pH 7,0 eingestellt und zweimal mit jeweils 2 l Ethylacetat extrahiert. Zur vollständigen Extraktion der Cytorhodine wird der pH der wäßrigen Phase auf 8,0 erhöht und diese mit 2 l Chloroform extrahiert. Die organischen Phasen werden über Natriumsulfat getrocknet und im Vakuum eingeengt. Man erhält folgende Produkte:

Fraktion I (pH 6,0): 11,4 g vorwiegend unpolare Cytorhodine
Fraktion II (pH 7,0): 8,3 g Cytorhodine mittlerer Polarität
Fraktion III (pH 8,0): 1,7 g polare Cytorhodine

4,8 g des getrockneten Extraktes aus der Fraktion II (pH 7,0) werden in 30 ml Methanol/1 % Triethylammoniumphosphatpuffer pH 7,0 = 93:7 (System A) gelöst und auf eine Stahlsäule mit 1,9 l RP-18 Kieselgel (LiChroprep RP-18, 25-40 µm, Merck), äquilibriert mit System A, aufgetragen und mit System A bei einem Fluß von 20 ml/min eluiert. Nach einem Vorlauf von 1000 ml werden Fraktionen à 20 ml aufgefangen und dünnschichtchromatographisch (Kieselgel 60 $F_{254}$ Merck, System B: Chloroform/Methanol/Eisessig/Wasser = 75:16:10:4) analysiert. Die Cytornodin Z enthaltenden Fraktionen (Fr. 55 - 95) werden vereinigt, mit dem gleichen Volumen Wasser versetzt und mit 1/5 des VolumensChloroform extrahiert. Die organische Phase wird mit Wasser gewaschen, über Natriumsulfat getrocknet, im Vakuum eingeengt und mit Hexan gefällt.
Ausbeute: 1,9 g angereichertes Cytorhodin Z.

**Beispiel 2**

1,5 g nach Beispiel 1 erhaltenes Cytorhodin Z werden in 6 ml Chloroform/Methanol/Eisessig/Wasser = 50:25:7:4 (System C) gelöst und auf eine Säule mit 205 ml Kieselgel 60 Merck 15-40 µm, äquilibiert mit System C, aufgetragen und mit System C eluiert. Nach 600 ml Vorlauf werden Fraktionen à 10 ml aufgefangen und dünnschichtchromatographisch (Kieselgel 60 $F_{254}$ Merck, System B) analysiert. Cytorhodin Z enthaltende Fraktionen (Fr. 10 - 60) werden vereinigt und bis zur Abscheidung der Chloroformphase mit 5 %iger wäßriger $Na_2HPO_4$-Lösung versetzt. Die organische Phase wird mit $Na_2PHO_4$-Lösung und mit Wasser gewaschen, über Natriumsulfat getrocknet, im Vakuum eingeengt und mit Hexan gefällt.
Ausbeute: 660 mg reines Cytorhodin Z.
Rote, amorphe Substanz, leicht löslich in Methanol, Ethylacetat, Chloroform, unlöslich in Wasser und Hexan.
Dünnschichtchromatographie: Kieselgel 60 $F_{254}$ Merck
System: $CHCl_3$ MeOH/Eisessig/$H_2O$ 75:16:10:4; $R_f$ = 0.50
UV-Maxima: 236, 256, 296, 466 (Sch.), 493, 512 (Sch.) in Methanol/10 % HCl
NMR-Spektrum: Fig. 1
$C_{48}H_{68}N_2O_{15}$, M ber. 912 (FAB-MS bestätigt)
Cytorhodin 2 besitzt die Formel I worin $R^1$ Roa-Rod-Rod und $R^2$ Roa bedeutet.

**Beispiel 3**

0,2 g nach Beispiel 2 hergestelltes Cytorhodin Z werden in 20 ml verdünnter Salzsäure (pH 1,3) 2 Stunden bei Raumtemperatur gerührt. Die Lösung wird mit einem Volumen Chloroform extrahiert, die wäßrige Phase mit 1 N NaOH auf pH 8,0 gestellt und zweimal mit je einem Volumen Chloroform extrahiert. Die organischen Phasen werden vereinigt, über Natriumsulfat getrocknet, im Vakuum eingeengt und mit Hexan gefällt.
Ausbeute: 135 mg Hydrolyseprodukt = 9-0,10-0-Bis (α-L-rhodosaminyl)-γ- rhodomycinon.

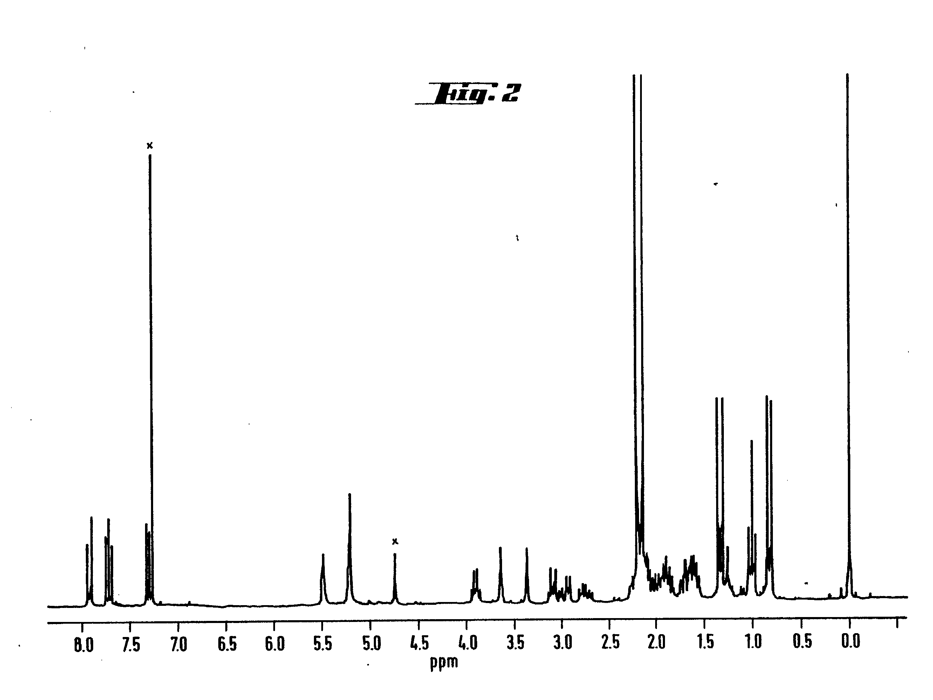

Rote, amorphe Substanz, leicht löslich in Methanol, Ethylacetat, Chloroform, unlöslich in Wasser und Hexan.
Dünnschichtchromatographie: Kieselgel 60 $F_{254}$ Merck
System B: Chloroform/Methanol/Eisessig/Wasser 75:16:10:4; $R_f$ = 0.27
UV-Maxima: 236, 255, 295, 465 (Sch.), 493, 513 (Sch.), in Methanol/10 % HCl
NMR-Spektrum: Fig. 2
$C_{36}H_{48}O_{11}$ $M_{ber.}$ 684 (FAB-MS bestätigt).

Die Identifizierung der Verbindungen aus den vorstehenden Beispielen erfolgt mit den nachstehend beschriebenen Meßbedingungen: die $^1$NMR-Spektren wurden auf einem HX-270 BRUKER Fourier Transform-Kernsonanz Spektrometer bei 270 MHz gemessen. Die Konzentrationen betrugen 2 - 4 mg/0,5 ml 99.8 %iges $CDCl_3$; die Lösungen wurden sofort nach Herstellung mit 0,1 ml 5 %iger $Na_2CO_3$ in 99,5 % $D_20$ geschüttelt.

Die in den Abbildungen mit einem Stern * versehenen Signale stammen von Lösungsmittelresten. Die Massenspektren wurden auf dem Massenspektrometer MS-902 S, EAI unter Verwendung einer FAB-Ionenquelle gemessen.

**Ansprüche**

1. Verbindungen der Formel I

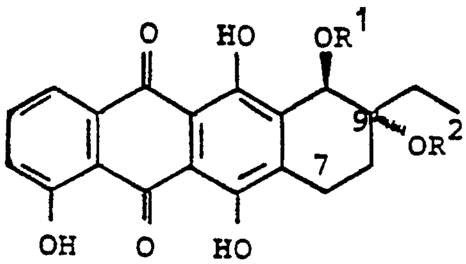

(I)

worin $R^1$ den Zuckerrest Roa oder die Zuckerkombination Roa-Rod-Rod und $R^2$ den Zuckerrest Roa bedeuten, sowie deren pharmakologisch unbedenkliche Salze.

2. Verfahren zur Herstellung von Verbindungen gemäß Anspruch 1, dadurch gekennzeichnet, daß man das Cytorhodin-Rohgemisch, das man gemäß EP-A-0 131 181 durch Kultivierung des Mikroorganismus-Stammes Y-11 472 (DSM 2658) und Extraktion des Mycels und des Kulturfiltrats mit organischen Lösungsmitteln erhält,

a) in einer wäßrigen Pufferlösung bei einem pH-Wert von 3,0 bis 4,0 löst, die erhaltene Lösung bei stufenweise erhöhten pH-Werten mit einem organischen Lösungsmittel extrahiert, und aus den aufkonzentrierten Extrakten mittels chromatographischer Reinigung die Verbindung der Formel I, worin $R^1$ Roa-Rod-Rod und $R^2$ Roa bedeutet, isoliert, und

b) gegebenenfalls die erhaltene Verbindung mit einer anorganischen Säure hydrolysiert zu der Verbindung der Formel I, worin $R^1$ und $R^2$ Roa bedeuten.

3. Verfahren nach Anspruch 2, dadurch gekennzeichnet, daß die wäßrige Pufferlösung gemäß Schritt a) stufenweise bei den pH-Werten 6,0, 7,0 und 8,0 mit Essigsäureethylester extrahiert wird.

4. Arzneimittel, gekennzeichnet durch einen Gehalt an einer Verbindung gemäß Anspruch 1.

5. Verwendung einer Verbindung gemäß Anspruch 1 zur Herstellung eines Arzneimittels mit cytostatischer Wirkung.

Patentansprüche: für folgende Vertragsstaaden: ES, GR

1. Verfahren zur Herstellung von Verbindungen der Formel I

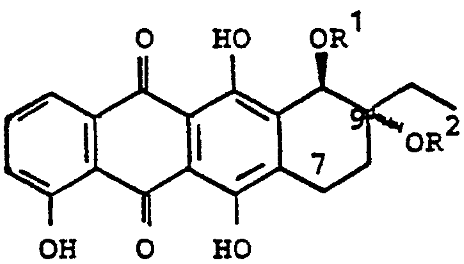

(I)

worin $R^1$ den Zuckerrest Roa oder die Zuckerkombination Roa-Rod-Rod und $R^2$ den Zuckerrest Roa bedeuten, sowie deren pharmakologisch unbedenkliche Salze, dadurch gekennzeichnet, daß man das Cytorhodin-Rohgemisch, das man gemäß EP-A-0 131 181 durch Kultivierung des Mikroorganismus-Stammes Y-11 472 (DSM 2658) und Extraktion des Mycels und des Kulturfiltrats mit organischen Lösungsmitteln erhält,

a) in einer wäßrigen Pufferlösung bei einem pH-Wert von 3,0 bis 4,0 löst, die erhaltene Lösung bei stufenweise erhöhten pH-Werten mit einem organischen Lösungsmittel extrahiert, und aus den aufkonzentrierten Extrakten mittels chromatographischer Reinigung die Verbindung der Formel I, worin $R^1$ Roa-Rod-Rod und $R^2$ Roa bedeutet, isoliert, und

b) gegebenenfalls die erhaltene Verbindung mit einer anorganischen Säure hydrolysiert zu der Verbindung der Formel I, worin $R^1$ und $R^2$ Roa bedeuten.

2. Verfahren nach Anspruch 1, dadurch gekennzeichnet, daß die wäßrige Pufferlösung gemäß Schritt a)

stufenweise bei den pH-Werten 6,0, 7,0 und 8,0 mit Essigsäureethylester extrahiert wird.

3. Verwendung einer Verbindung gemäß Anspruch 1 zur Herstellung eines Arzneimittels mit cytostatischer Wirkung.

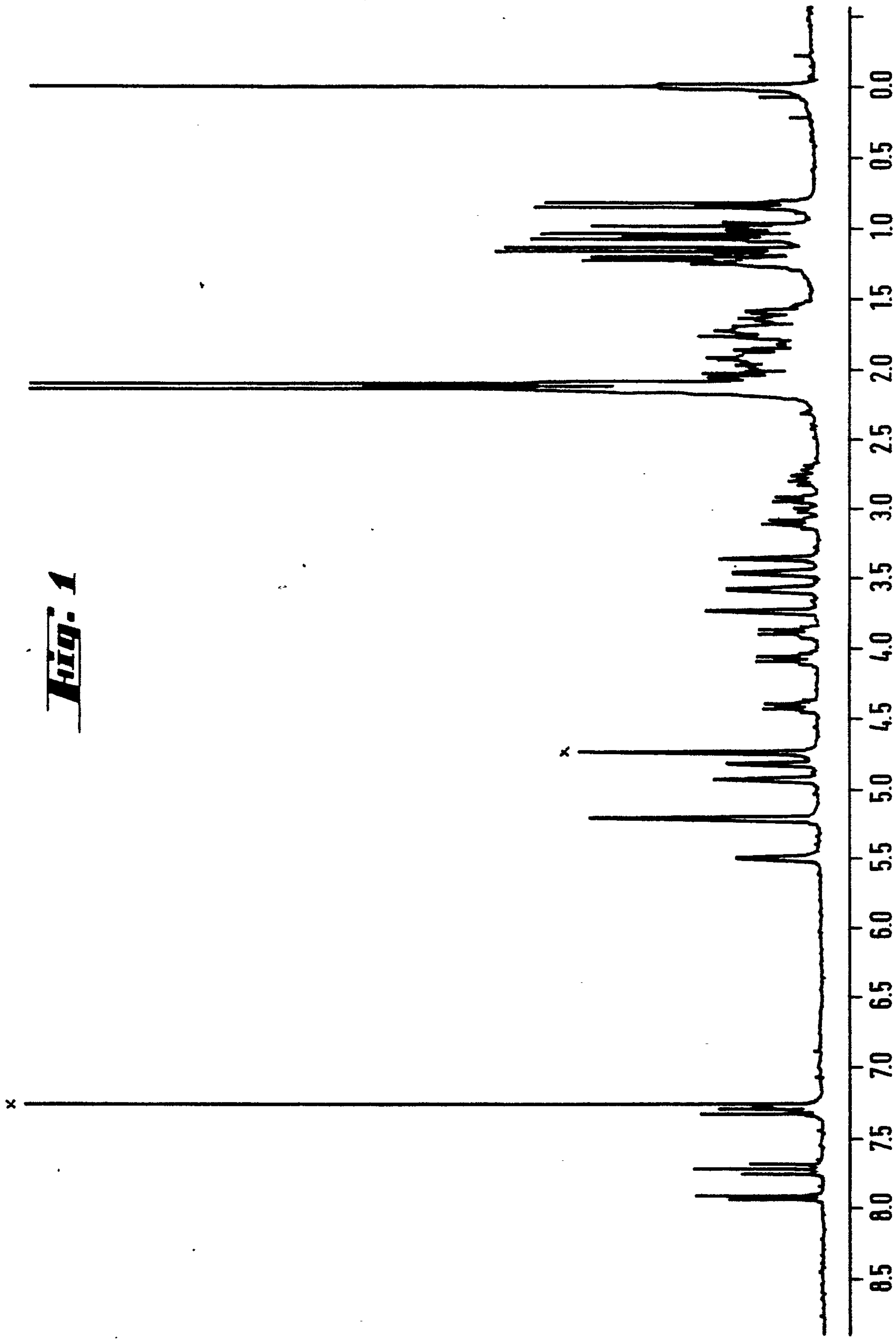


Fig. 1

Fig. 2